(19) Europäisches Patentamt
European Patent Office
Office européen des brevets

(11) Veröffentlichungsnummer: **0 457 090 B1**

(12) # EUROPÄISCHE PATENTSCHRIFT

(45) Veröffentlichungstag der Patentschrift: **29.11.95**

(51) Int. Cl.[6]: **C07C 229/34**, C07D 213/30, C07C 255/30, C07C 243/18, C07C 227/14, C07D 215/56

(21) Anmeldenummer: **91106962.3**

(22) Anmeldetag: **30.04.91**

(54) **Verfahren zur Herstellung von 3-Amino-2-(het)aroyl-acrylsäurederivaten.**

(30) Priorität: **12.05.90 DE 4015299**

(43) Veröffentlichungstag der Anmeldung:
**21.11.91 Patentblatt 91/47**

(45) Bekanntmachung des Hinweises auf die Patenterteilung:
**29.11.95 Patentblatt 95/48**

(84) Benannte Vertragsstaaten:
**AT BE CH DE DK ES FR GB GR IT LI LU NL SE**

(56) Entgegenhaltungen:
**EP-A- 0 176 846**
**EP-A- 0 300 311**

**JOURNAL FüR PRAKTISCHE CHEMIE Bd. 330, Nr. 6, 1988, LEIPZIG Seiten 981 - 992; G.W. FISCHER: 'Modifizierung von (1-Aryl-1H-tetrazol-5-yl)-acetaldehyd-Enaminen durch säurekatalysierte Umaminierung'**

**WORLD PATENTS INDEX LATEST Week 8714, Derwent Publications Ltd., London, GB; AN 87-097756 & JP-A-62 045 580**

**JOURNAL FüR PRAKTISCHE CHEMIE Bd. 332, Nr. 6, Juni 1990, LEIPZIG Seiten 977 - 994; G.W. FISCHER: 'Synthese und Umaminierung von Enaminoketonen der Tetrazolreihe'**

(73) Patentinhaber: **BAYER AG**

**D-51368 Leverkusen (DE)**

(72) Erfinder: **Grohe, Klaus, Dr.**
**Am Wasserturm 10**
**W-5068 Odenthal (DE)**

EP 0 457 090 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von 3-Amino-2-(het)aroyl-acrylsäurederivaten, die wertvolle Zwischenprodukte für die Synthese von hochwirksamen antibakteriellen Arzneimitteln sind.

Es ist bekannt, daß man am Stickstoffatom monosubstituierte 3-Amino-2-benzoylacrylsäurederivate erhält, wenn man 3-Dialkylamino-2-benzoylacrylsäurederivate mit primären aliphatischen Aminen in inerten organischen Lösungsmitteln wie z.B. Cyclohexan, Toluol, Chlorbenzol oder Butylglykol bei höherer Temperatur umsetzt (EP 176846, EP 300311). Ein Nachteil dieses Verfahrens ist, daß das bei der Umsetzung freigesetzte Dialkylamin unter den Reaktionsbedingungen in einer Nebenreaktion kernständige, reaktionsfähige Halogenatome angreift. Es können dann für die Folgereaktionen schwer trennbare und daher ungeeignete Substanzgemische erhalten werden.

Aus Vestnik beloruskogo gosudarstvennogo universiteta, ISSN 0372-5340, 1986, Nr. 1, Seiten 67 - 69 ist bekanntgeworden, daß 2-Morpholino-cycloalkenone mit primären Aminen in Gegenwart von Essigsäure umaminiert werden können. Aus dem Journal für praktische Chemie, Bd. 330, Heft 6, 1988, Seiten 981-992 ist bekanntgeworden, daß sekundäre Tetrazolylvinylamine sich durch Säure katalysierte Umaminierung aus 5-(2-Dimethylamino-vinyl)-1H-tetrazolen herstellen lassen. Offensichtlich dissoziieren Aminsalze unter den dort beschriebenen Reaktionsbedingungen hinreichend stark, um als solche reagieren zu können, so daß nicht zu erwarten ist, daß unerwünschte Reaktionen freien Amins durch die Gegenwart von Säure verhindert werden können.

Es wurde nun gefunden, daß man 3-Amino-2-(het)aroyl-acrylsäurederivate der Formel (I),

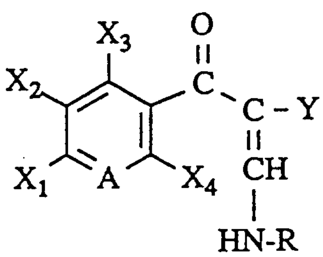

(I)

in welcher

- Y für eine Nitril- oder Estergruppe -COOR$^1$ sowie eine Acetylgruppe steht, wobei
- $R^1$ $C_1$-$C_4$-Alkyl bedeutet und
- R Alkyl mit 1 bis 6 Kohlenstoffatomen, 2-Fluorethyl, 2-Chlorethyl, 2-Hydroxyethyl, 1-(Hydroxymethyl)-ethyl, Cyclopropyl, Methoxy, 4-Fluorphenyl, 2,4-Difluorphenyl, Dimethylamino, Formyl-methylamino, Isopropylidenamino sein kann,
- A für Stickstoff oder C-R$^2$ steht, wobei
- $R^2$ Wasserstoff, Methyl, Halogen, Nitro, Methoxy oder Cyano sein kann,
- $X^1$ Halogen, bevorzugt Chlor oder Fluor sein kann und
- $X^2$ für Halogen, bevorzugt Fluor, steht sowie
- $X^3$ Wasserstoff, Halogen oder Nitro bedeutet und
- $X^4$ Halogen, bevorzugt Chlor oder Fluor, Nitro, Methoxy oder Methylthiol sein kann,

erhält, wenn man 3-Dialkylamino-2-(het)aroyl-acrylsäurederivate der Formel (II),

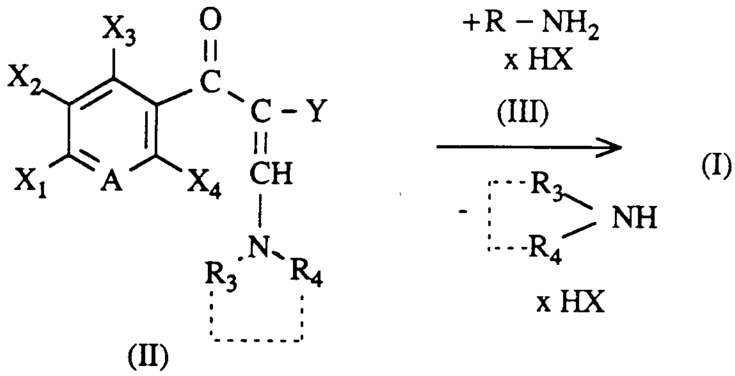


(II)

PP25

in welcher
A, $X^1$-$X^4$ und Y die oben angegebene Bedeutung haben und $R^3$ bzw. $R^4$ die gleich oder verschieden sein können und für eine Alkylgruppe mit 1 bis 4 Kohlenstoffatomen stehen oder zusammen mit dem Stickstoffatom, an das sie gebunden sind, einen 5- bzw. 6-gliedrigen Ring bilden, der zusätzlich die Atome oder Gruppen -O-, -S- oder -$SO_2$- enthalten kann, mit primären Aminen (III), in welchen R die oben angegebene Bedeutung besitzt in Gegenwart von zumindest einem Äquivalent einer Säure in einem Lösungsmittel oder in überschüssiger Säure umsetzt.

Es muß als ausgesprochen überraschend gelten, daß die 3-Dialkylamino-2(het)aroyl-acrylsäurederivate (II) mit den Salzen (III) zu (I) reagieren.

Ein besonderer Vorteil dieses neuen Verfahrens ist, daß im (Het)arylkern von (II) vorhandene reaktionsfähige Halogenatome nicht mit den Aminsalzen reagieren. Man erhält daher unmittelbar reine, für die Folgereaktionen geeignete Zwischenprodukte (I).

Verwendet man den 3-Dimethylamino-2-(2,3,4,5-tetrafluorbenzoyl)-acrylsäureethylester (1), Cyclopropylamin (2) und Essigsäure als Ausgangsstoffe, so kann der erfindungsgemäße Reaktionsablauf durch das folgende Formelschema wiedergegeben werden:

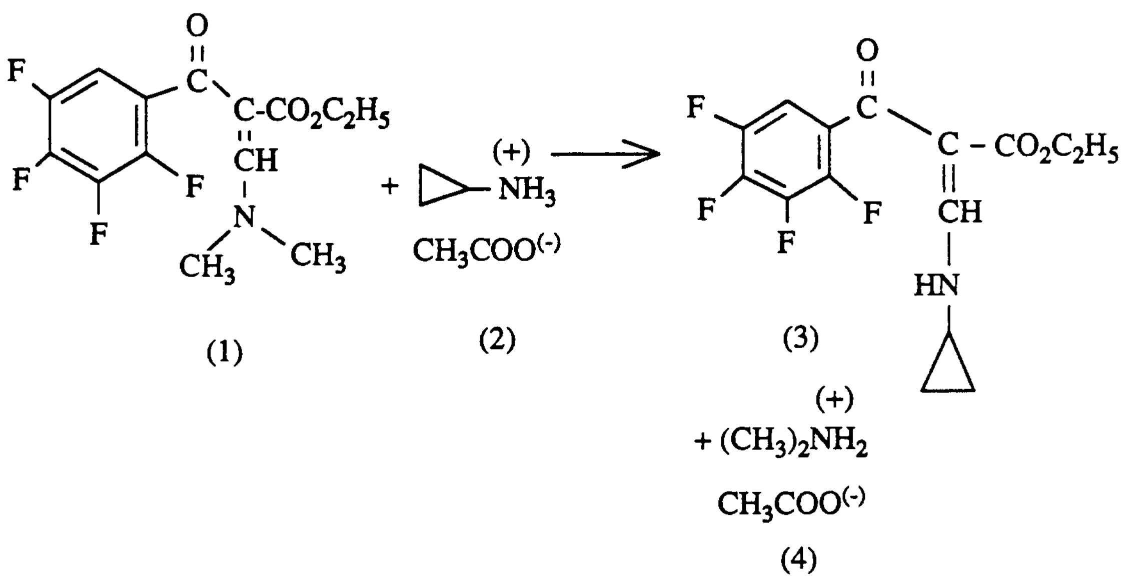

(1) (2) (3) (4)

Das bei der Umsetzung von (1) mit (2) zu (3) gebildete Dimethylamin wird als essigsaures Satz (4) abgefangen. Eine Reaktion mit dem aktivierten 2- bzw. 4-ständigen Fluoratom von (1) bzw. (3) wird so vermieden.

Bei der nachfolgenden Cyclisierung von (3) entsteht der Chinoloncarbonester (5), der zur Säure (6) verseift wird und nach Austausch des 7-ständigen Fluoratoms gegen sekundäre Aminreste die antibakteriell hochwirksamen 7-Amino-chinoloncarbonsäuren (7) liefert.

(3) 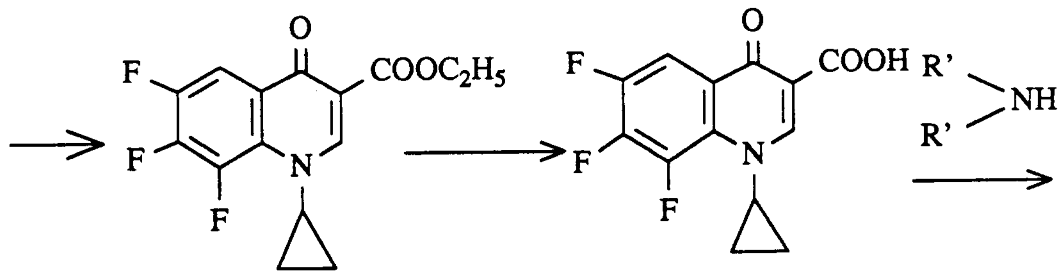

(5) (6)

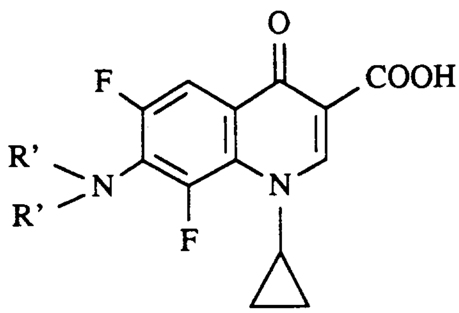

(7)

Die erfindungsgemäß verwendbaren Amine III sind bekannt. Als Beispiele seien genannt: Methylamin, Ethylamin, 2-Fluorethylamin, 2-Hydroxyethylamin, Isopropylamin, tert.-Butylamin, Cyclopropylamin, O-Methylhydroxylamin, 4-Fluoranilin, 2,4-Difluoranilin, 1-Formyl-1-methylhydrazin, 1,1-Dimethylhydrazin.

Gleichfalls bekannt oder nach bekannten Verfahren leicht herstellbar sind die Acrylsäurederivate II (EP 176 846, EP 300 311). Beispielhaft seien genannt: 3-Dimethylamino-2-(2,4,5-trifluorbenzoyl)-acrylsäureethylester, 3-Dimethylamino-2-(2,4-dichlor-5-fluorbenzoyl)-acrylsäureethylester, 3-Dimethylamino-2-(2,4-dichlor-5-fluorbenzoyl)-acrylnitril, 3-Pyrrolidino-2-(2,4-dichlor-5-fluorbenzoyl)-acrylsäuremethylester, 3-Dimethylamino-2-(2,3,4,5-tetrafluorbenzoyl)-acrylsäureethylester, 3-Dimethylamino-2-(2,3,4,5-tetrafluorbenzoyl)-acrylnitril, 3-Dimethylamino-2-(2,4-dichlor-5-fluor-3-nitrobenzoyl)-acrylsäureethylester, 3-Dimethylamino-2-(2,3,4,5,6-pentafluorbenzoyl)-acrylsäureethylester, 3-Dimethylamino-2-(3-chlor-2,4,5-trifluorbenzoyl)-acrylsäureethylester, 3-Dimethylamino-2-(2,4-dichlor-3,6-difluorbenzoyl)-acrylsäureethylester, 3-Diethylamino-2-(2,3,4,5-tetrafluorbenzoyl)-acrylsäureethylester` 3-Morpholino-2-(2,4-dichlor-5-fluorbenzoyl)-acrylsäuremethylester, 3-Dimethylamino-2-(2,6-dichlor-5-fluornicotinyl)-acrylsäureethylester,3-Dimethylamino-2-(2,6-dichlor-5-fluornicotinyl)-acrylnitril, 3-Dimethylamino-2-(2,6-dichlornicotinyl)-acrylsäureethylester.

Beim erfindungsgemäßen Verfahren wird II mit einem Satz einer organischen oder anorganischen Säure von III in einem organischen Lösungs- oder Verdünnungsmittel umgesetzt. Als Verdünnungsmittel kann man beispielsweise Essigsäure, Propionsäure, Glykolsäure, Toluol, Chloroform, Dioxan, Dimethylsulfoxid, N-Methylpyrrolidon, Dimethylformamid oder Tetramethylharnstoff verwenden.

Als organische bzw. anorganische Säure kommen beispielsweise Essigsäure, Propionsäure, Glykolsäure, Salzsäure, Methansulfonsäure, p-Toluolsulfonsäure, Milchsäure, Zitronensäure, Weinsäure, Äpfelsäure, Bernsteinsäure, Maleinsäure oder Fumarsäure in Frage, die alleine oder im Gemisch eingesetzt werden können.

Die Umsetzung wird bei Temperaturen zwischen 10 °C und 150 °C, vorzugsweise zwischen 20 °C und 100 °C, durchgeführt. Es wird bevorzugt bei Normaldruck gearbeitet.

Die Reaktionspartner II und III werden im Molverhältnis 1:1 bis 1:4, vorzugsweise 1:1 bis 1:1,1, eingesetzt.

Bei der Umsetzung von II mit III zu I können mehrere Verfahrensvarianten angewandt werden:

a) Der Dialkylaminoacrylester oder das entsprechende Nitril II wird in Essigsäure vorgelegt und das Amin III tropfen- bzw. portionsweise zugegeben.

b) Zur Lösung oder Suspension von II in Essigsäure gibt man portionsweise ein Satz des Amins III oder tropfenweise eine Lösung dieses Satzes in Essigsäure.

c) Das Amin III wird zur Essigsäure getropft und anschließend eine Lösung von II in Essigsäure zugetropft bzw. II portionsweise eingetragen. Man kann auch ein Satz des Amins III mit einer anderen Säure in Essigsäure vorlegen.

d) Zur Lösung oder Suspension von II in einem inerten Verdünnungsmittel wie z.B. Toluol gibt man portionsweise das entsprechende Salz des Amins III.

e) Das Aminsalz wird in einem inerten Verdünnungsmittel wie z.B. Toluol vorgelegt und II portionsweise oder in Toluol gelöst tropfenweise zugegeben.

Es wird 0,5 Stunden bis 48 Stunden, vorzugsweise 2 bis 12 Stunden bei 10 °C bis 150 °C, vorzugsweise bei 20 °C bis 100 °C, gerührt.

Zur Aufarbeitung wird das Verdünnungsmittel im Vakuum abdestilliert, der Rückstand in Methylenchlorid/Wasser aufgenommen, die organische Phase mit Wasser gewaschen, mit Natriumsulfat getrocknet und das Methylenchlorid abdestilliert.

Wird ein mit Wasser mischbares Verdünnungsmittel wie z.B. Essigsäure, Dioxan oder Ethanol verwendet, so kann das Reaktionsgemisch auch auf Eis gegossen, der Niederschlag abgesaugt und getrocknet werden.

Die Rohprodukte von I werden entweder direkt weiter umgesetzt oder durch Umkristallisation zunächst gereinigt.

Beispiel 1

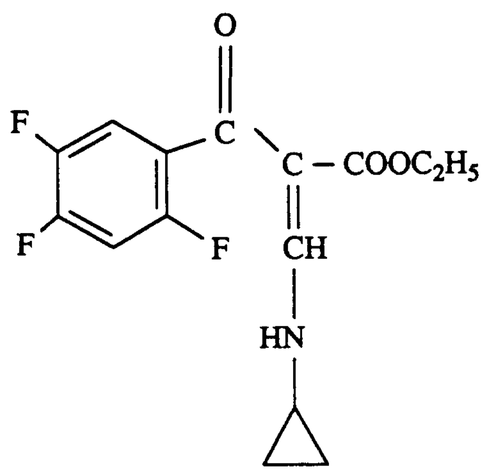

a) Eine Lösung von 29 g 3-Dimethylamino-2-(2,4,5-trifluorbenzoyl)-acrylsäureethylester in 100 ml Eisessig wird unter Rühren bei 10 °C bis 15 °C tropfenweise mit der Lösung von 6,3 g Cyclopropylamin in 40 ml Eisessig versetzt. Man rührt 6 Stunden bei Raumtemperatur und erwärmt dann 2 Stunden auf 50 °C bis 60 °C. Das Lösungsmittel wird dann im Vakuum abdestilliert, der Rückstand in Methylenchlorid/Wasser aufgenommen, mit Wasser und gesättigter $NaHCO_3$-Lösung gewaschen, über Natriumsulfat getrocknet und das Methylenchlorid abdestilliert.

Rohausbeute: 27,5 g (91,2 %) 3-Cyclopropylamino-2-(2,4,5-trifluorbenzoyl)-acrylsäureethylester vom Schmelzpunkt 54 °C bis 56 °C.

b) Zu 200 ml Eisessig tropft man zunächst unter Rühren bei ca. 10 °C 34,7 g Cyclopropylamin und dann eine Lösung von 182,7 g 3-Dimethylamino-2-(2,4,5-trifluorbenzoyl)acrylsäureethylester in 100 ml Eisessig. Man rührt 12 Stunden bei Raumtemperatur und 2 Stunden bei 50 °C bis 60 °C. Die Aufarbeitung erfolgt wie unter (a) beschrieben.

Ausbeute: 171 g (90 %) 3-Cyclopropylamino-(2,4,5-trifluorbenzoyl)-acrylsäureethylester vom Schmelzpunkt 54 °C bis 56 °C.

Beispiel 2

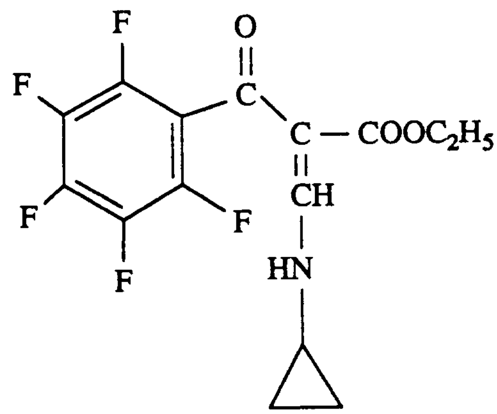

Zu 10 ml Eisessig tropft man unter Kühlung mit kaltem Wasser zunächst 1,3 g Cyclopropylamin und dann eine Lösung von 6,74 g 3-Dimethylamino-2-(2,3,4,5,6-pentafluorbenzoyl)-acrylsäureethylester in 25 ml Eisessig. Man rührt 2 Stunden bei Raumtemperatur und 2 Stunden bei 50 °C bis 60 °C. Die Aufarbeitung erfolgt wie unter (1a).
Ausbeute: 6,8 g (97,4 %) 3-Cyclopropylamino-2-(2,3,4,5,6-pentafluorbenzoyl)-acrylsäureethylester; Schmelzpunkt einer Probe nach Umkristallisation aus Cyclohexan/Leichtbenzin: 84 °C bis 85 °C.

Beispiel 3

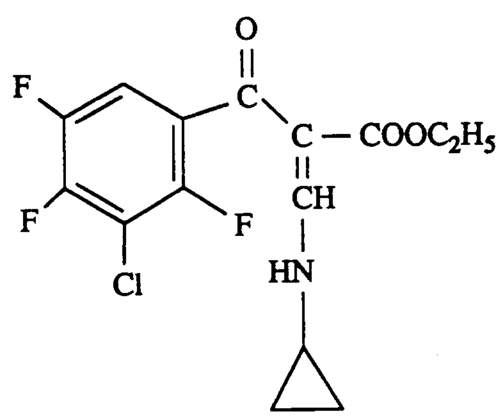

Zu 11 ml Eisessig tropft man unter Kühlung mit kaltem Wasser und Rühren 1,41 g Cyclopropylamin und dann eine Lösung von 7,3 g 3-Dimethylamino-2-(3-chlor-2,4,5-trifluorbenzoyl)-acrylsäure-ethylester in 25 ml Eisessig. Man rührt 12 Stunden bei Raumtemperatur und dann 2 Stunden bei 50-60 °C. Die Aufarbeitung erfolgt wie unter (1a) angegeben.
Ausbeute: 7,3 g 3-Cyclopropylamino-2-(3-chlor-2,4,5-trifluorbenzoyl)-acrylsäureethylester vom Schmelzpunkt 78-79 °C (nach Umkristallisation aus Ethanol/Wasser).

Beispiel 4

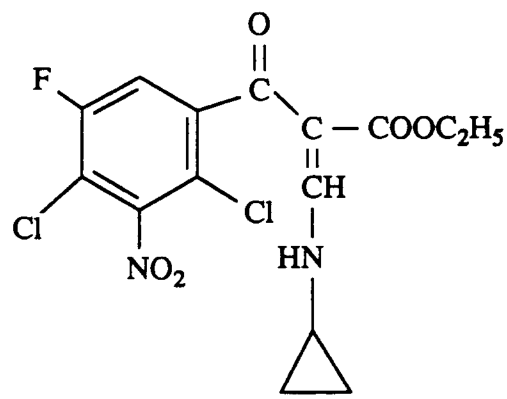

a) Zu 150 ml Eisessig tropft man unter Rühren und Kühlen mit kaltem Wasser 6,6 g Cyclopropylamin. Anschließend versetzt man portionsweise mit 37,9 g 3-Dimethylamino-2-(2,4-dichlor-5-fluor-3-nitrobenzoyl)-acrylsäureethylester. Man rührt 12 Stunden bei Raumtemperatur und dann 1 Stunde bei 50 °C bis 60 °C. Die heiße Lösung wird auf Eis gegossen, der Niederschlag abgesaugt, mit Wasser gewaschen und getrocknet.

Ausbeute: 36,5 g (93,3 %) 3-Cyclopropylamino-2-(2,4-dichlor-5-fluor-3-nitrobenzoyl)-acrylsäureethylester. Probe nach Umkristallisation aus Ethanol/Wasser vom Schmelzpunkt 146 °C bis 147 °C.

b) Zu einer Lösung von 1,25 g Cyclopropylamin in 30 ml Toluol tropft man unter Eiskühlung und Rühren zunächst 1,5 g Eisessig und dann eine Lösung von 7,4 g 3-Dimethylamino-2-(2,4-dichlor-5-fluor-3-nitrobenzoyl)-acrylsäureethylester in 30 ml Toluol. Man rührt 1 Stunde bei Raumtemperatur und 2 Stunden bei 70 °C bis 80 °C. Die erkaltete Lösung wird mit Wasser und Natriumbicarbonat-Lösung gewaschen, mit Natriumsulfat getrocknet und das Lösungsmittel im Vakuum abdestilliert.

Ausbeute: 7,5 g (98 %) 3-Cyclopropylamino-2-(2,4-dichlor-5-fluor-3-nitrobenzoyl)-acrylsäureethylester vom Schmelzpunkt 139 °C bis 141 °C.

Beispiel 5

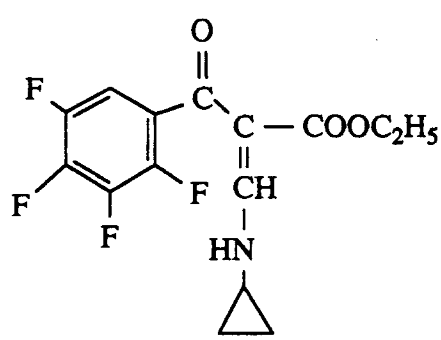

Zu 50 ml Eisessig tropft man unter Rühren und Kühlen mit kaltem Wasser 6 g Cyclopropylamin und dann eine Lösung von 29,2 g 3-Dimethylamino-2-(2,3,4,5-tetrafluorbenzoyl)-acrylsäureethylester. Man rührt 6 Stunden bei Raumtemperatur 2 Stunden bei 50 °C bis 60 °C und destilliert darauf das Lösungsmittel im Vakuum ab. Die Aufarbeitung erfolgt wie unter (1a) beschrieben.

Ausbeute: 29,7 g (98 %) 3-Cyclopropylamino-2-(2,3,4,5-tetrafluorbenzoyl)-acrylsäureethylester vom Schmelzpunkt 64 °C. Nach Umkristallisation aus Cyclohexan/Leichtbenzin beträgt der Schmelzpunkt 66 °C bis 68 °C.

Beispiel 6

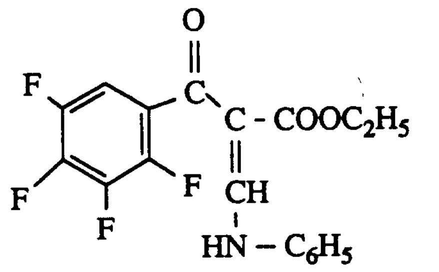

Zu 10 ml Eisessig tropft man unter Rühren und Kühlen mit kaltem Wasser 3,3 g Anilin sowie eine Lösung von 10 g 3-Dimethylamino-2-(2,3,4,5-tetrafluorbenzoyl)-acrylsäureethylester in 60 ml Eisessig. Man rührt 2 Stunden bei Raumtemperatur, 2 Stunden bei 70 ° C bis 80 ° C und arbeitet wie unter (1a) beschrieben auf.

Rohausbeute: 10 g (86,9 %) 3-Anilino-2-(2,3,4,5-tetrafluorbenzoyl)-acrylsäureethylester. Probe aus Cyclohexan umkristallisiert. Schmelzpunkt: 91 ° C.

Beispiel 7

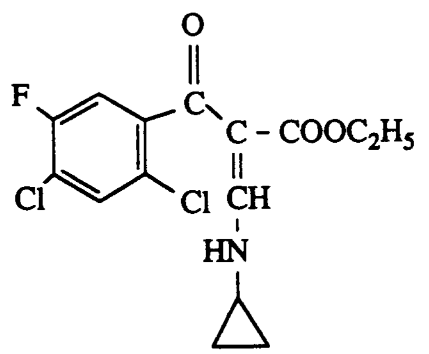

a) Eine Lösung von 6,6 g 3-Dimethylamino-2-(2,4-dichlor-5-fluorbenzoyl)-acrylsäureethylester in 40 ml Eisessig wird bei ca. 10 ° C unter Rühren tropfenweise mit 1,35 g Cyclopropylamin versetzt. Man rührt 12 Stunden bei Raumtemperatur oder 30 Minuten bei Raumtemperatur und 1,5 Stunden bei 80 ° C und arbeitet nach Beispiel (1a) auf.

Ausbeute: 6,7 g (97,9 %) 3-Cyclopropylamino-2-(2,4-dichlor-5-fluorbenzoyl)-acrylsäureethylester vom Schmelzpunkt 87 ° C bis 88 ° C.

b) Eine Lösung von 1,5 g Eisessig in 60 ml Chloroform wird tropfenweise mit 1,35 g Cyclopropylamin versetzt. Dann gibt man portionsweise 6,6 g 3-Dimethylamino-2-(2,4-dichlor-5-fluorbenzoyl)-acrylsäureethylester zu, rührt 1 Stunde bei Raumtemperatur und erhitzt 3 Stunden unter Rückfluß. Das Reaktionsgemisch wird dann mit Wasser und Natriumbicarbonat-Lösung gewaschen, die organische Phase mit Natriumsulfat getrocknet und das Lösungsmittel abdestilliert.

Ausbeute: 6,5 g (95 %) 3-Cyclopropylamino-2-(2,4-dichlor-5-fluorbenzoyl)-acrylsäureethylester vom Schmelzpunkt 84 ° C bis 86 ° C.

c) Zu einer Lösung von 0,63 g Cyclopropylamin in 30 ml Toluol tropft man unter Eiskühlung und Rühren 1,1 g Methansulfonsäure. Dann versetzt man portionsweise mit 2,8 g 3-Dimethylamino-2-(2,4-dichlor-5-fluorbenzoyl)-acrylsäureethylester, rührt 1 Stunde bei Raumtemperatur und 2 Stunden bei 70 ° C bis 80 ° C. Die Suspension wird mit Wasser und Natriumbicarbonat-Lösung gewaschen, mit Natriumsulfat getrocknet und das Lösungsmittel im Vakuum abdestilliert.

Ausbeute: 2,9 g (96,5 %) 3-Cyclopropylamino-2-(2,4-dichlor-5-fluorbenzoyl)-acrylsäureethylester vom Schmelzpunkt 88 ° C bis 89 ° C.

Beispiel 8

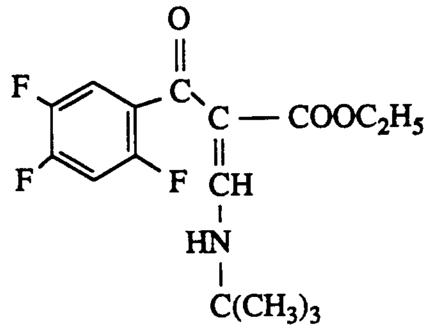

Bei ca. 10 ° C werden 50 ml Eisessig unter Rühren tropfenweise mit 8 g tert.-Butylamin versetzt. Dann tropft man eine Lösung von 32 g 3-Dimethylamino-2-(2,4,5-trifluorbenzoyl)-acrylsäureethylester in 80 ml Eisessig zu, rührt 12 Stunden bei Raumtempeatur und arbeitet wie bei Beispiel (1a) beschrieben auf.
Rohausbeute: 28,5 g (81,5 %) 3-tert.-Butylamino-2-(2,4,5-trifluorbenzoyl)-acrylsäureethylester. Probe aus Cyclohexan/Leichtbenzin umkristallisiert. Schmelzpunkt 100 ° C bis 101 ° C.

Beispiel 9

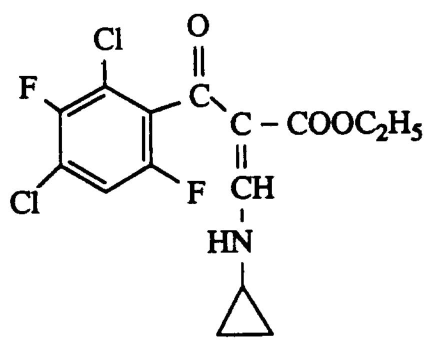

Zu 140 ml Eisessig tropft man unter Rühren und Kühlen mit kaltem Wasser 18,8 g Cyclopropylamin und anschließend eine Lösung von 105 g 3-Dimethylamino-2-(2,4-dichlor-3,6-difluorbenzoyl)-acrylsäureethylester in 130 ml Eisessig. Es wird 12 Stunden bei Raumtemperatur, 2 Stunden bei 50 ° C bis 60 ° C gerührt und wie bei Beispiel (1a) aufgearbeitet.
Ausbeute: 83 g (76,4 %) 3-Cyclopropylamino-2-(2,4-dichlor-3,6-difluorbenzoyl)-acrylsäureethylester vom Schmelzpunkt 98 ° C bis 100 ° C.

Beispiel 10

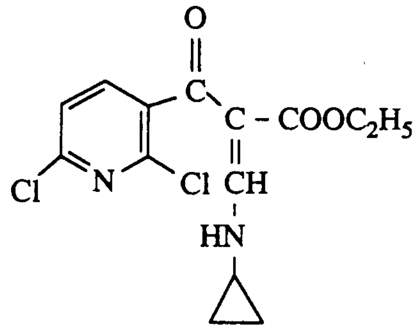

Analog Beispiel (4a) setzt man 3,17 g 3-Dimethylamino-2-(2,6-dichlornicotinyl)-acrylsäureethylester mit 0,6 g Cyclopropylamin in Eisessig um und erhält den 3-Cyclopropylamino-2-(2,6-dichlornicotinyl)-acrylsäureethylester vom Schmelzpunkt 131 ° C bis 133 ° C in einer Ausbeute von 94 % der Theorie.

Beispiel 11

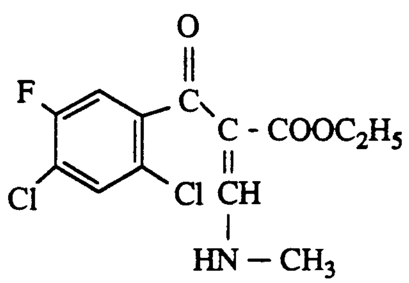

Eine Lösung von 3,3 g 3-Dimethylamino-2-(2,4-dichlor-5-fluorbenzoyl)-acrylsäureethylester in 30 ml Toluol versetzt man mit 0,74 g Methylaminhydrochlorid, rührt 1 Stunde bei Raumtemperatur und 4 Stunden bei 70 ° C bis 80 ° C. Die Aufarbeitung erfolgt wie bei Beispiel (7c).
Ausbeute: 3,0 g (94,9 %) 3-Methylamino-2-(2,4-dichlor-5-fluorbenzoyl)-acrylsäureethylester vom Schmelzpunkt 95 ° C bis 97 ° C.

Nach dem erfindungsgemäßen Verfahren lassen sich auch die Beispiele 12 bis 37 analog den Beispielen 1 bis 11 in vergleichbaren Ausbeuten herstellen.

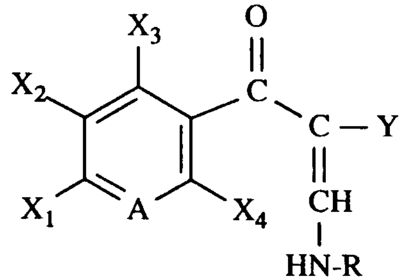

(I)

| Bsp. Nr. | $X^1$ | $X^2$ | $X^3$ | $X^4$ | A | Y | R | Schmelzpunkt [°C] |
|---|---|---|---|---|---|---|---|---|
| 12 | Cl | F | H | Cl | CH | $COOC_2H_5$ | $C(CH_3)_3$ | 91-92 |
| 13 | Cl | F | H | Cl | CH | $COOCH_3$ | 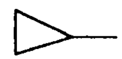 | 146-148 |
| 14 | Cl | F | H | Cl | CH | CN | 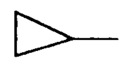 | 93-95 |
| 15 | Cl | F | H | $CH_3O$ | CH | $COOCH_3$ | 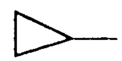 | 130-132 |
| 16 | Cl | F | H | Cl | CH | CN | 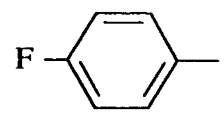 | 195-197 |
| 17 | F | F | H | F | CF | $COOC_2H_5$ | 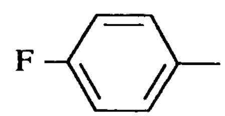 | 93-95 |

| Bsp. Nr. | $X^1$ | $X^2$ | $X^3$ | $X^4$ | A | Y | R | Schmelzpunkt [°C] |
|---|---|---|---|---|---|---|---|---|
| 18 | F | F | H | F | CF | $COOC_2H_5$ | 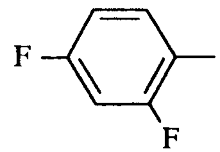 | 114-116 |
| 19 | Cl | F | H | Cl | C-$NO_2$ | $COO_2H_5$ | 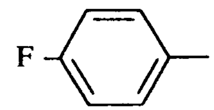 | 129-131 |
| 20 | Cl | F | H | Cl | C-$NO_2$ | $COOCH_3$ | 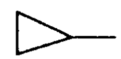 | 168-169 |
| 21 | Cl | F | H | Cl | C-$NO_2$ | $COOC_2H_5$ | $FCH_2CH_2$ | 123-124 |
| 22 | Cl | F | H | Cl | CH | $COOC_2H_5$ | 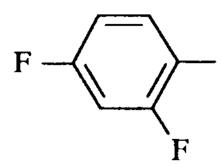 | 102-104 |
| 23 | F | F | H | F | CF | $COOC_2H_5$ | $CH_3$ | 155-157 |

| Bsp. Nr. | $X^1$ | $X^2$ | $X^3$ | $X^4$ | A | Y | R | Schmelzpunkt [°C] |
|---|---|---|---|---|---|---|---|---|
| 24 | F | F | H | F | CH | $COOCH_3$ | 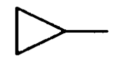 | 123-125 |
| 25 | Cl | F | H | Cl | CH | $COOC_2H_2$ | $(CH_3)_2CH$- | 97- 98 |
| 26 | F | F | H | F | CH | $COOC_2H_5$ | 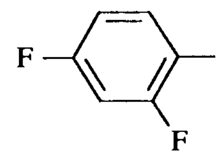 | 107-109 |
| 27 | Cl | F | Cl | F | CH | $COOC_2H_5$ | 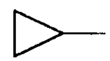 | 106-108 |
| 28 | Cl | F | Cl | F | CH | $COOC_2H_5$ | 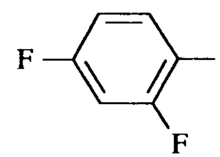 | 91- 93 |
| 29 | F | F | H | F | C-Cl | $COOC_2H_5$ | 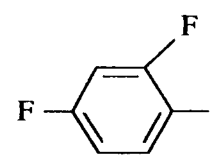 | 128-130 |

| Bsp. Nr. | $X^1$ | $X^2$ | $X^3$ | $X^4$ | A | Y | R | Schmelzpunkt [°C] |
|---|---|---|---|---|---|---|---|---|
| 30 | F | F | F | F | C-F | $COOC_2H_5$ | 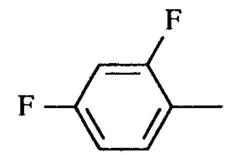 | 99-101 |
| 31 | Cl | F | H | Cl | C-H | $COOC_2H_5$ | $HOCH_2CH_2$- | 90- 92 |
| 32 | F | F | H | F | C-F | $COOC_2H_5$ | $CH_3CH_2$- | 115-117 |
| 33 | F | F | H | F | C-F | $COOC_2H_5$ | $CH_3O$- | 152-153 |
| 34 | Cl | F | H | Cl | $C-NO_2$ | $COOC_2H_5$ | $CH_3CH_2$- | 143-145 |
| 35 | Cl | | H | Cl | N | $COOC_2H_5$ | 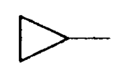 | 131-133 |

| Bsp. Nr. | $X^1$ | $X^2$ | $X^3$ | $X^4$ | A | Y | R | Schmelzpunkt [°C] |
|---|---|---|---|---|---|---|---|---|
| 36 | Cl | F | H | Cl | N | $COOC_2H_5$ | 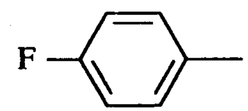 | 112-114 |
| 37 | Cl | F | H | Cl | N | $COOC_2H_5$ | 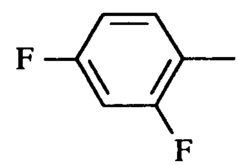 | 138-140 |

Beispiel 38

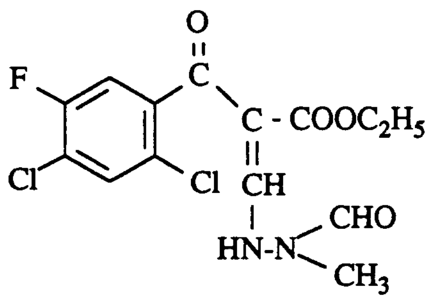

Zu 30 ml Eisessig tropft man unter Rühren und Kühlen mit kaltem Wasser 0,82 g 1-Formyl-1-methylhydrazin. Dann gibt man 3,3 g 3-Dimethylamino-2-(2,4-dichlor-5-fluorbenzoyl)-acrylsäureethylester portionsweise zu, rührt 1,5 Stunden bei Raumtemperatur, 2 Stunden bei 50 °C bis 60 °C und destilliert das Lösungsmittel im Vakuum ab. Die Aufarbeitung geschieht wie unter Beispiel (1a) beschrieben.
Ausbeute: 2,9 g (80,9 %) 3-(2-Formyl-2-methylhydrazino)-2-(2,4-dichlor-5-fluorbenzoyl)-acrylsäureethylester vom Schmelzpunkt 104 °C bis 106 °C (nach Umkrisstallisation aus Cyclohexan/Leichtbenzin).

A) Eisessig-Verfahren

Beispiel 39 (Vgl. Beispiel 5)

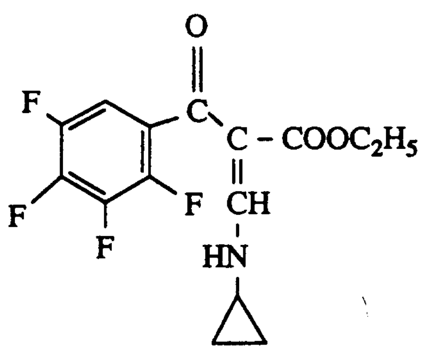

Zu 50 ml Eisessig tropft man unter Rühren und Kühlen mit kaltem Wasser 1,2 g Cyclopropylamin. Dann versetzt man portionsweise mit 6,38 g 3-Dimethylamino-2-(2,3,4,5-tetrafluorbenzoyl)-acrylsäureethylester und spült mit ca. 10 ml Ethanol nach. Man rührt 6 Stunden bei Raumtemperatur, läßt über Nacht bei Raumtemperatur stehen und destilliert die Lösungsmittel im Vakuum ab. Die Aufarbeitung erfolgt wie unter (1a) beschrieben.
Ausbeute: 6 g vom Schmelzpunkt 57 bis 59 °C.

Cyclisierung zum Chinoloncarbonsäureester:

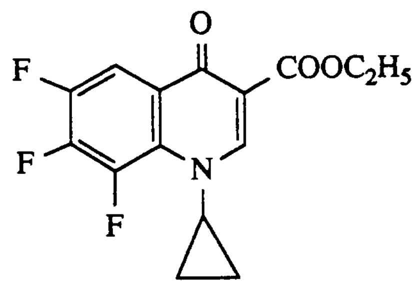

3,31 g (0,01 Mol) 3-Cyclopropylamino-2-(2,3,4,5-tetrafluorbenzoyl)-acrylsäureethylester, 0,65 g Natriumfluorid und 20 ml N-Methylpyrrolidon werden 3 Stunden auf 140 °C erhitzt. Man gießt in 100 ml Wasser, saugt den Niederschlag ab und wäscht gut mit Wasser nach. Nach dem Trocknen im Vakuum bei 100 °C erhält man 2,8 g (90 %) 1-Cyclopropyl-6,7,8-trifluor-1,4-dihydro-4-oxo-chinolincarbonsäureethylester vom Schmelzpunkt 156 bis 159 °C. Nach dem in Chloroform aufgenommenem $^1$H-NMR-Spektrum enthält das Produkt kein 7-Dimethylamino-1-cyclopropyl-6,8-difluor-1,4-dihydro-oxo-chinolincarbonsäure ethylester.

B) Toluol-Verfahren

Beispiel 40 (Vergleichsbeispiel)

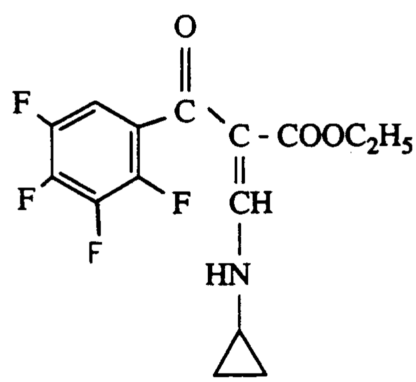

6,38 g (0,02 Mol) 3-Dimethylamino-2-(2,3,4,5-tetrafluorbenzoyl)-acrylsäureethylester werden mit 1,2 g Cyclopropylamin und 30 ml Toluol bis zur Beendigung der Gasentwicklung (ca. 1 Stunde) unter Rückfluß zum Sieden erhitzt. Dann wird das Lösungsmittel im Vakuum abdestilliert und wie bei (1a) aufgearbeitet. Ausbeute: 5,9 g vom Schmelzpunkt 56 bis 58 °C.

Cyclisierung zum Chinoloncarbonester:

3,31 g (0,01 Mol) nach dem Toluol-Verfahren erhaltener 3-Cyclopropylamino-2-(2,3,4,5-tetrafluorbenzoyl)-acrylsäureethylester, 0,65 g Natriumfluorid und 20 ml N-Methylpyrrolidon werden 3 Stunden auf 140 °C erhitzt. Man gießt in 100 ml Wasser, saugt den Niederschlag ab und wäscht gut mit Wasser nach. Nach dem Trocknen im Vakuum bei 100 °C erhält man 2,8 g (90 %) 1-Cyclopropyl-6,7,8-trifluor-1,4-dihydro-4-oxo-chinolincarbonsäureethylester vom Schmelzpunkt 149 bis 152 °C. Nach dem in Chloroform aufgenommenem $^1$H-NMR-Spektrum enthält das Produkt ca. 7 Mol-% 1-Cyclopropyl-7-dimethylamino-6,8-difluor-1,4-dihydro-4-oxo-chinolincarbonsäureethylester.

## Patentansprüche

**1.** Verfahren zur Herstellung von 3-Amino-2-(het)aroyl-acrylsäurederivaten der Formel (I),

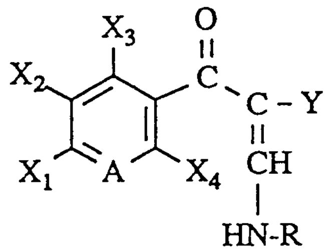

(I)

in welcher

| | |
|---|---|
| Y | für eine Nitril- oder Estergruppe -COOR[1] sowie eine Acetylgruppe steht, wobei |
| $R^1$ | $C_1$-$C_4$-Alkyl bedeutet und |
| R | Alkyl mit 1 bis 6 Kohlenstoffatomen, 2-Fluorethyl, 2-Chlorethyl, 2-Hydroxyethyl, 1-(Hydroxymethyl)-ethyl, Cyclopropyl, Methoxy, 4-Fluorphenyl, 2,4-Difluorphenyl, Dimethylamino, Formyl-methylamino, Isopropylidenamino bedeutet, |
| A | für Stickstoff oder C-$R^2$ steht, wobei |
| $R^2$ | Wasserstoff, Methyl, Halogen, Nitro, Methoxy oder Cyano bedeuten, |
| $X^1$ und $X^2$ | gleich oder verschieden ist und Halogen bedeuten, sowie |
| $X^3$ | Wasserstoff, Halogen oder Nitro und |
| $X^4$ | Halogen, Nitro, Methoxy oder Methylthiol bedeutet, |

dadurch gekennzeichnet, daß man 3-Dialkylamino-2-(het)aroyl-acrylsäurederivate der Formel (II),

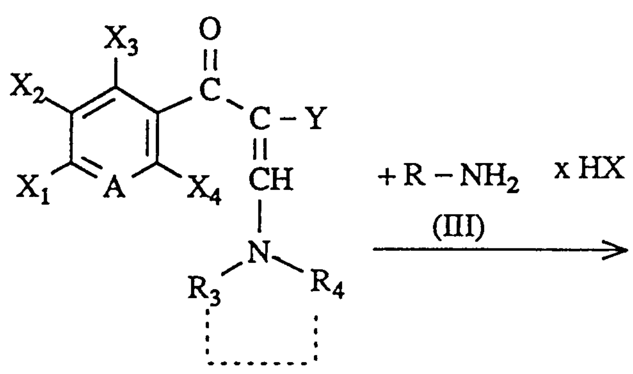


(I)

(II)

in welcher

A, $X^1$-$X^4$ und Y die oben angegebene Bedeutung haben und $R^3$ bzw. $R^4$ die gleich oder verschieden sein können und für eine Alkylgruppe mit 1 bis 4 Kohlenstoffatomen stehen oder zusammen mit dem Stickstoffatom, an das sie gebunden sind, einen 5- bzw. 6-gliedrigen Ring bilden, der zusätzlich die Atome oder Gruppen -O-, -S- oder $-SO_2-$ enthalten kann,
mit primären Aminen (III) in welchen R die oben angegebene Bedeutung besitzt in Gegenwart von zumindest einem Äquivalent einer Säure HX in einem Lösungsmittel oder in überschüssiger Säure umsetzt.

**2.** Verfahren gemäß Anspruch 1, dadurch gekennzeichnet, daß bei den 3-Amino-2-(het)aroyl-acrylsäurederivaten der Formel (I),

| | |
|---|---|
| Y | für eine Nitril- oder Estergruppe -COOR[1] sowie eine Acetylgruppe steht, wobei |
| $R^1$ | $C_1$-$C_4$-Alkyl bedeutet und |

R Alkyl mit 1 bis 6 Kohlenstoffatomen, 2-Fluorethyl, 2-Chlorethyl, 2-Hydroxyethyl, 1-(Hydroxymethyl)-ethyl, Cyclopropyl, Methoxy, 4-Fluorphenyl, 2,4-Difluorphenyl, Dimethylamino, Formyl-methylamino, Isopropylidenamino bedeutet,

A für Stickstoff oder C-$R^2$ steht, wobei

$R^2$ Wasserstoff, Methyl, Halogen, Nitro, Methoxy oder Cyano bedeuten,

$X^1$ Chlor oder Fluor und

$X^2$ Fluor bedeutet

$X^3$ Wasserstoff, Halogen oder Nitro bedeutet und

$X^4$ Chlor oder Fluor, Nitro, Methoxy oder Methylthiol bedeutet.

**3.** Verfahren gemäß Anspruch 1, dadurch gekennzeichnet, daß man die Umsetzung bei Temperaturen von 10 °C bis 150 °C durchführt.

**4.** Verfahren gemäß Anspruch 1, dadurch gekennzeichnet, daß das Molverhältnis der Säure HX zum Amin R-$NH_2$ 1:1 bis 4:1 ist.

**5.** Verfahren gemäß Anspruch 1, dadurch gekennzeichnet, daß es sich bei der Säure HX um eine organische bzw. anorganische Säure handelt.

## Claims

**1.** Process for the preparation of 3-amino-2-(het)aroyl-acrylic acid derivatives of the formula (I)

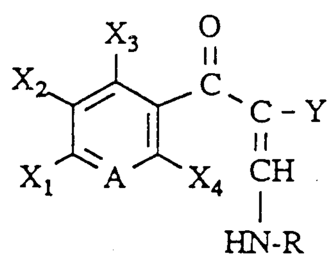

(I)

in which

Y represents a nitrile or ester group -$COOR^1$ and also an acetyl group, where

$R^1$ denotes $C_1$-$C_4$-alkyl and

R denotes alkyl having 1 to 6 carbon atoms, 2-fluoroethyl, 2-chloroethyl, 2-hydroxyethyl, 1-(hydroxymethyl)-ethyl, cyclopropyl, methoxy, 4-fluorophenyl, 2,4-difluorophenyl, dimethylamino, formyl-methylamino or isopropylideneamino,

A represents nitrogen or C-$R^2$, where

$R^2$ denotes hydrogen, methyl, halogen, nitro, methoxy or cyano,

$X^1$ and $X^2$ are identical or different and denote halogen, and

$X^3$ denotes hydrogen, halogen or nitro and

$X^4$ denotes halogen, nitro, methoxy or methylthiol,

characterised in that 3-dialkylamino-2-(het)aroyl-acrylic acid derivatives of the formula (II)

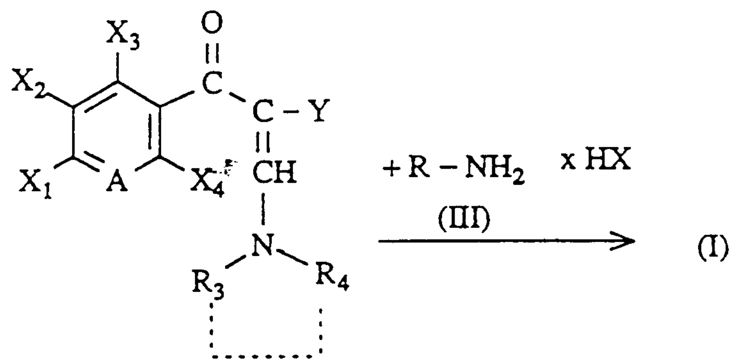

(II)

in which

A, $X^1$-$X^4$ and Y have the abovementioned meaning and $R^3$ and $R^4$ can be the same or different and represent an alkyl group having 1 to 4 carbon atoms or, together with the nitrogen atom to which they are bonded, form a 5- or 6-membered ring which additionally can contain the atoms or groups -O-, -S- or $-SO_2-$, are reacted with primary amines (III), in which R has the abovementioned meaning, in the presence of at least one equivalent of an acid HX in a solvent or in excess acid.

**2.** Process according to Claim 1, characterised in that, in the 3-amino-2-(het)aroyl-acrylic acid derivatives of the formula (I),

- Y represents a nitrile or ester group $-COOR^1$ and also an acetyl group, where
- $R^1$ denotes $C_1$-$C_4$-alkyl and
- R denotes alkyl having 1 to 6 carbon atoms, 2-fluoroethyl, 2-chloroethyl, 2-hydroxyethyl, 1-(hydroxymethyl)-ethyl, cyclopropyl, methoxy, 4-fluorophenyl, 2,4-difluorophenyl, dimethylamino, formyl-methylamino or isopropylideneamino,
- A represents nitrogen or C-$R^2$, where
- $R^2$ denotes hydrogen, methyl, halogen, nitro, methoxy or cyano,
- $X^1$ denotes chlorine or fluorine and
- $X^2$ denotes fluorine, and
- $X^3$ denotes hydrogen, halogen or nitro and
- $X^4$ denotes chlorine or fluorine, nitro, methoxy or methylthiol.

**3.** Process according to Claim 1, characterised in that the reaction is carried out at temperatures from 10 °C to 150 °C.

**4.** Process according to Claim 1, characterised in that the molar ratio of the acid HX to the amine $R-NH_2$ is 1:1 to 4:1.

**5.** Process according to Claim 1, characterised in that the acid HX is an organic or inorganic acid.

## Revendications

**1.** Procédé pour la préparation de dérivés d'acides 3-amino-2-(het)aroyl-acryliques répondant à la formule (I)

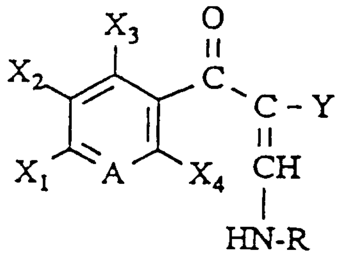

(I) .

dans laquelle

| | |
|---|---|
| Y | représente un groupe nitrile ou un groupe ester, $-COOR^1$, ainsi qu'un groupe acétyle, où |
| $R^1$ | représente un groupe alkyle en $C_1$-$C_4$ et |
| R | représente un groupe alkyle contenant de 1 à 6 atomes de carbone, un groupe 2-fluoréthyle, un groupe 2-chloréthyle, un groupe 2-hydroxyéthyle, un groupe 1-(hydroxyméthyl)-éthyle, un groupe cyclopropyle, un groupe méthoxy, un groupe 4-fluorophényle, un groupe 2,4-difluorophényle, un groupe diméthylamino, un groupe formylméthylamino, un groupe isopropylidènamino, |
| A | représente un atome d'azote ou $C-R^2$, où |
| $R^2$ | représente un atome d'hydrogène, un groupe méthyle, un atome d'halogène, un groupe nitro, un groupe méthoxy ou un groupe cyano, |
| $X^1$ et $X^2$ | sont identiques ou différents et représentent un atome d'halogène, et |
| $X^3$ | représente un atome d'hydrogène, un atome d'halogène ou un groupe nitro, et |
| $X^4$ | représente un atome d'halogène, un groupe nitro, un groupe méthoxy ou un groupe méthylthiol, |

caractérisé en ce qu'on fait réagir des dérivés d'acides 3-dialkylamino-2-(het)aroyl-acryliques de formule (II)

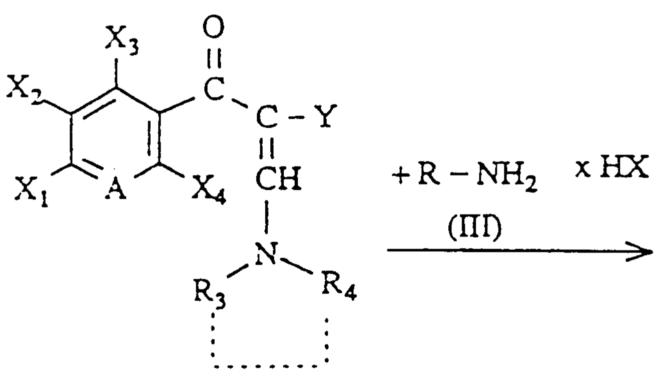


(I)

(II)

dans laquelle

| | |
|---|---|
| A, $X^1$-$X^4$ et Y | ont la signification indiquée ci-dessus, et $R^3$, respectivement $R^4$ peuvent être identiques ou différents et représentent un groupe alkyle contenant de 1 à 4 atomes de carbone, ou représentent, ensemble avec l'atome d'azote auquel ils sont liés, un noyau penta-, respectivement hexagonal, qui peut contenir, en outre, les atomes ou les groupes -O-, -S- ou $-SO_2-$, avec des amines primaires (III) dans lesquelles R possède la signification indiquée ci-dessus, en présence d'au moins un équivalent d'un acide HX dans un solvant ou dans de l'acide en excès. |

2. Procédé selon la revendication 1, caractérisé en ce que, dans les dérivés d'acides 3-amino-2-(het)-aroylacryliques de formule (I),
   - Y représente un groupe nitrile ou un groupe ester, $-COOR^1$, ainsi qu'un groupe acétyle, où
   - $R^1$ représente un groupe alkyle en $C_1$-$C_4$ et
   - R représente un groupe alkyle contenant de 1 à 6 atomes de carbone, un groupe 2-fluoréthyle, un groupe 2-chloréthyle, un groupe 2-hydroxyéthyle, un groupe 1-(hydroxyméthyl)-éthyle, un groupe cyclopropyle, un groupe méthoxy, un groupe 4-fluorophényle, un groupe 2,4-difluorophényle, un groupe diméthylamino, un groupe formylméthylamino, un groupe isopropylidènamino,
   - A représente un atome d'azote ou C-$R^2$, où
   - $R^2$ représente un atome d'hydrogène, un groupe méthyle, un atome d'halogène, un groupe nitro, un groupe méthoxy ou un groupe cyano,
   - $X^1$ représente un atome de chlore ou un atome de fluor et
   - $X^2$ représente un atome de fluor,
   - $X^3$ représente un atome d'hydrogène, un atome d'halogène ou un groupe nitro, et
   - $X^4$ représente un atome de chlore ou un atome de fluor, un groupe nitro, un groupe méthoxy ou un groupe méthylthiol.

3. Procédé selon la revendication 1, caractérisé en ce qu'on effectue la mise en réaction à des températures de 10 °C à 150 °C.

4. Procédé selon la revendication 1, caractérisé en ce que le rapport molaire de l'acide HX à l'amine R-$NH_2$ est de 1:1 à 4:1.

5. Procédé selon la revendication 1, caractérisé en ce que, en ce qui concerne l'acide HX, il s'agit d'un acide organique, respectivement inorganique.